# EUROPEAN PATENT SPECIFICATION

(11) **EP 3 205 370 B1**
(45) Date of publication and mention of the grant of the patent: **28.08.2019**
(21) Application number: 15849116.7
(22) Date of filing: 17.09.2015
(51) Int. Cl.: A61M 25/10, A61B 5/0408, A61B 5/0478, A61B 5/0492, A61B 17/00, A61M 31/00, A61B 5/042, A61N 1/06, A61B 18/00

(54) **CHEMICAL ABLATION DEVICE AND CHEMICAL ABLATION SYSTEM**
CHEMISCHE ABLATIONSVORRICHTUNG UND CHEMISCHES ABLATIONSSYSTEM
DISPOSITIF D'ABLATION CHIMIQUE ET SYSTÈME D'ABLATION CHIMIQUE

(30) Priority: 08.10.2014 JP 2014206899
(43) Date of publication of application: 16.08.2017
(73) Proprietor: Japan Lifeline Co., Ltd., Tokyo 140-0002 (JP)
(72) Inventor: MITSUMUNE, Norihiko, Tokyo 140-0002 (JP); MORI, Kenji, Tokyo 140-0002 (JP)
(74) Representative: Grünecker Patent- und Rechtsanwälte PartG mbB
(86) International application number: PCT/JP2015/076593
(87) International publication number: WO 2016/056376

(56) References cited:
- EP-A1- 2 022 397
- WO-A2-01/37723
- JP-A- H09 499
- JP-A- H1 176 183
- JP-A- 2002 301 163
- JP-A- 2002 500 644
- JP-A- 2004 081 798
- JP-A- 2005 348 947
- JP-A- 2006 247 344
- JP-A- 2007 190 428
- JP-A- 2008 245 767
- JP-A- 2008 504 067
- JP-A- 2013 202 068
- JP-A- 2014 036 856
- JP-A- 2014 524 342
- US-A- 4 957 110
- US-A- 5 464 394
- US-A- 5 669 881
- US-B1- 7 341 571

## Description

### Technical Field

The present invention relates to a chemical ablation device and a chemical ablation system that are suitably applicable to, for example, a method of ethanol infusion in the vein of Marshall.

### Background Art

A chemical ablation method of necrotizing arrhythmogenic tissues by way of ethanol infusion in the vein of Marshal (a method of ethanol infusion in the vein of Marshall) has been attracting attention as a catheter treatment for atrial fibrillation (see NPL 1 listed below).

Here, the vein of Marshal (VOM) is a vein branching off from the coronary sinus and running from the rear wall toward the side wall of the left atrium on the side of the epicardium.

### Citation List

US 5,669,881 discloses a vascular introducer comprising a pliable tube having an occlusion balloon mounted thereon to occlude or block blood flow through a blood vessel into which the introduced is inserted. From EP 2 022 397, an ablation device, including a catheter and an ablation element incorporating one or more balloons is known. From WO 01/37723, a probe facilitating creation of circumferential lesions in body tissue is known.

US-7,341,571-B1, US-5,464,394-A and US-4,957,110-A show further prior art devices.

### Non Patent Literature

NPL 1: Heart Rhythem. 2009 November; 6(11): 1552-1558 (Ethanol Infusion in Vein of Marshall: Adjuctive Effects during Ablation of Atrial Fibrillation)

### Summary of Invention

### Technical Problem

In a method of ethanol infusion in the vein of Marshall, steps (1) to (9) given below may be performed with a chemical ablation system that includes a primary guiding catheter, a secondary guiding catheter insertable into a lumen of the primary guiding catheter, an electrode catheter for measuring (pacing and/or mapping) the potential in the vein of Marshall, and a balloon catheter that ejects ethanol from a distal tip thereof.

Here, the electrode catheter and the balloon catheter included in the system are inserted into and removed from the vein of Marshall along a guidewire. Therefore, the electrode catheter has a cylindrical shape with a guidewire lumen. Furthermore, the balloon catheter has an over-the-wire double-lumen structure with a guidewire lumen and an expansion lumen.

### (1) Insertion of Primary Guiding Catheter:

First, approaching from the superior vena cava, the distal end of the primary guiding catheter is made to engage with the entrance of the coronary sinus.

### (2) Insertion of Secondary Guiding Catheter:

Subsequently, the secondary guiding catheter is inserted into the lumen of the primary guiding catheter, and the distal end of the secondary guiding catheter that is made to project from an opening at the distal end of the primary guiding catheter is positioned near the entrance of the vein of Marshall.

### (3) Insertion of Guidewire:

Subsequently, the guidewire is inserted into the lumen of the secondary guiding catheter, and a distal portion of the guidewire that is made to project from an opening at the distal end of the secondary guiding catheter is inserted into the vein of Marshall. This operation is usually performed under fluoroscopy with view of an X-ray image.

### (4) Insertion of Electrode Catheter (Potential Measurement):

Subsequently, the electrode catheter having a cylindrical shape is inserted into the lumen of the secondary guiding catheter along the guidewire, and a distal portion thereof to which an electrode is attached is made to project from the opening at the distal end of the secondary guiding catheter and is inserted into the vein of Marshall. Then, pre-treatment potential measurement (pacing/mapping) is performed.

### (5) Removal of Electrode Catheter and Insertion of Balloon Catheter:

Subsequently, the electrode catheter is removed along the guidewire, the over-the-wire balloon catheter is inserted into the lumen of the secondary guiding catheter along the guidewire, and a distal portion thereof that is made to project from the opening at the distal end of the secondary guiding catheter is inserted into the vein of Marshall.

### (6) Removal of Guidewire and Ablation Treatment:

Subsequently, the guidewire that is lying in the guidewire lumen of the balloon catheter is removed, and the balloon is expanded. Then, ethanol is infused into the guidewire lumen from a hemostasis valve connected to the proximal end of the balloon catheter. The ethanol thus infused flows through the guidewire lumen and the lumen at the distal tip of the balloon catheter and is infused from the opening of the distal tip into the vein of Marshall, whereby chemical ablation is performed. Usually, chemical ablation (ethanol infusion) starts to be performed from the far side where capillary vessels are present, then chemical ablation is performed multiple times (in three to four times) by moving the balloon catheter stepwise towards the entrance.

### (7) Re-Insertion of Guidewire:

Subsequently, the guidewire removed at the time of ablation treatment is re-inserted into the vein of Marshall along the guidewire lumen of the balloon catheter.

### (8) Removal of Balloon Catheter:

Subsequently, the balloon catheter is removed along the re-inserted guidewire.

### (9) Re-Insertion of Electrode Catheter (Potential Measurement):

Subsequently, the electrode catheter used in the above step (4) is inserted into the lumen of the primary guiding catheter along the guidewire, and the distal portion thereof to which the electrode is attached is re-inserted into the vein of Marshall, whereby post-treatment potential measurement (pacing/matching) is performed.

Here, if the result of the potential measurement shows that the ablation is not satisfactory, the above steps (5) to (9) are performed again.

However, such a procedure (ablation treatment including pre- and post-treatment potential measurements) employing the above chemical ablation system is very complicated.

The present invention is conceived with consideration for the above circumstances.

An object of the present invention is to provide a chemical ablation device and a chemical ablation system that enable simple performance of chemical ablation treatment including pre- and post-treatment potential measurements.

Another object of the present invention is to provide a chemical ablation device and a chemical ablation system that enable simple performance of a procedure associated with a method of ethanol infusion in the vein of Marshall.

### Solution to Problem

A chemical ablation device according to the present invention includes:
an electrode-equipped guidewire with which intracardiac potential is measurable;
an over-the-wire balloon catheter including an inner tube having a guidewire lumen into which the electrode-equipped guidewire is to be inserted, an outer tube provided on an outer side of the inner tube in such a manner as to provide an expansion lumen, a balloon provided on the outer side of the inner tube with a proximal end thereof attached to a distal end of the outer tube, and a distal tip that is continuous with the inner tube and extends from a distal end of the balloon;
a Y-shaped connector connected to a proximal side of the balloon catheter and including a guidewire port communicating with the guidewire lumen of the balloon catheter, and an expansion port communicating with the expansion lumen of the balloon catheter; and
a hemostasis valve connected to the guidewire port of the Y-shaped connector and including a side-infusion tube for supplying a medical fluid for ablation to the guidewire lumen of the balloon catheter,
wherein the medical fluid supplied to the guidewire lumen of the balloon catheter is ejected from an opening of the distal tip.

In the chemical ablation device configured as above, the electrode-equipped guidewire included therein has both a function of a guidewire and a function of an electrode catheter that are provided to known devices, and the medical fluid is supplied to the guidewire lumen of the balloon catheter from the side-infusion tube included in the hemostasis valve connected to the guidewire port of the Y-shaped connector. Therefore, when the medical fluid is ejected to a site of interest from the opening of the distal tip through the guidewire lumen and the lumen of the distal tip in which the electrode-equipped guidewire is lying, treatment can be performed with the electrode-equipped guidewire dwelling in the body. Hence, the post-treatment potential measurement can be performed immediately. If the measured potential shows that the treatment is insufficient, the treatment can be restarted immediately.

Accordingly, there is no need to perform a complicated operation employed in the known devices, including the removal, at the time of treatment, of the electrode catheter having been inserted for pre-treatment potential measurement (in step (5) given above); the removal of the guidewire at the time of treatment (in step (6) given above); and the re-insertion of the guidewire, the removal of the balloon catheter, and the re-insertion of the electrode catheter for post-treatment potential measurement (in steps (7) to (9) given above).

In the chemical ablation device according to the present invention, it is preferable that the electrode-equipped guidewire include a core wire, a resin shaft that encloses at least a distal portion of the core wire excluding a distalmost part of the core wire, a connector connected to a proximal side of the resin shaft with or without a metal shaft in between, a plurality of ring-shaped electrodes each provided around an outer periphery of the resin shaft, and a plurality of conductor wires connected to the ring-shaped electrodes, respectively, and to the connector while running through an inside of the resin shaft.

Furthermore, it is preferable that the electrode-equipped guidewire include a coil spring attached to the distalmost part of the core wire and extending in an axial direction of the core wire.

Furthermore, it is preferable that the electrode-equipped guidewire is provided with an electrode on a distal side of the distalmost part of the core wire with respect to a position where the coil spring is provided.

Furthermore, it is preferable that an outside diameter of the electrode provided on the distalmost part of the core wire of the electrode-equipped guidewire and an outside coil diameter of the coil spring of the electrode-equipped guidewire be each 0.76 mm or smaller, and
that the resin shaft have an outside diameter of 0.89 mm or smaller.

Furthermore, it is preferable that a difference between an opening diameter of the distal tip included in the balloon catheter and the outside diameter of the resin shaft included in the electrode-equipped guidewire be 0.05 to 0.50 mm, and
that, in a state where the distal portion of the resin shaft is made to project from the opening of the distal tip, the medical fluid be ejected from a gap between the distal tip and the resin shaft.

In the chemical ablation device according to the present invention, it is preferable that the distal tip included in the balloon catheter have a plurality of side holes on an outer periphery thereof.

In the chemical ablation device configured as above, the medical fluid can be supplied to a site that is on the proximal side with respect to the opening of the distal tip.

A chemical ablation system according to the present invention includes a primary guiding catheter having an inside diameter of 1.9 to 2.6 mm, a secondary guiding catheter that is insertable into a lumen of the primary guiding catheter and has an inside diameter of 1.5 to 2.2 mm, and the chemical ablation device according to the present invention.

### Advantageous Effects of Invention

The chemical ablation device and the chemical ablation system according to the present invention enable simple performance of chemical ablation treatment including pre-and post-treatment potential measurements, in particular, a procedure associated with a method of ethanol infusion in the vein of Marshall.

### Brief Description of Drawings

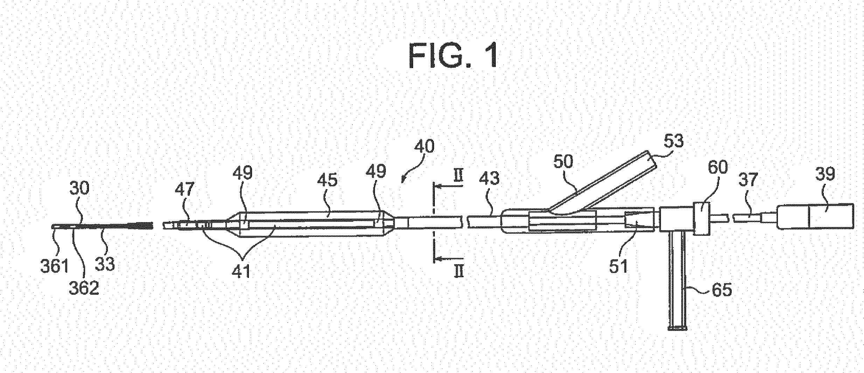

Fig. 1 is a side view of an exemplary chemical ablation device according to the present invention.

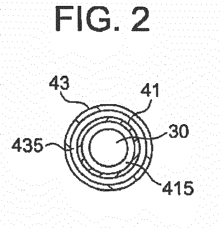

Fig. 2 is a II-II sectional view of Fig. 1.

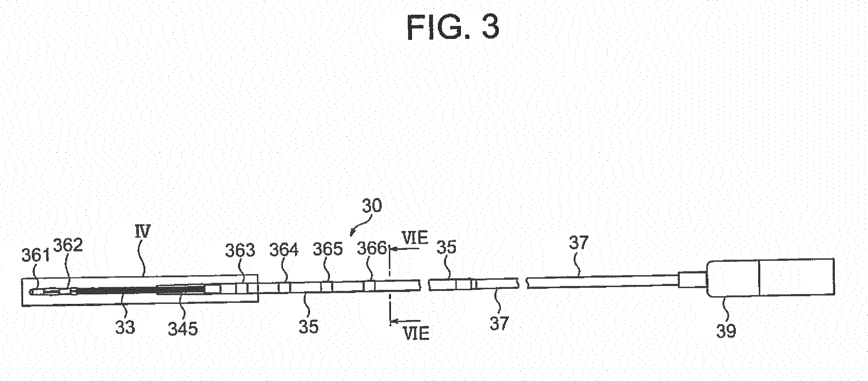

Fig. 3 is a side view of an electrode-equipped guidewire included in the chemical ablation device illustrated in Fig. 1.
Fig. 4 is an enlargement of a part (a detailed diagram of part IV) illustrated in Fig. 3.
Fig. 5 is a V-V sectional view of Fig. 4.
Fig. 6A is a VIA-VIA sectional view of Fig. 4.
Fig. 6B is a VIB-VIB sectional view of Fig. 4.
Fig. 6C is a VIC-VIC sectional view of Fig. 4.
Fig. 6D is a VID-VID sectional view of Fig. 4.
Fig. 6E is a VIE-VIE sectional view of Fig. 3.

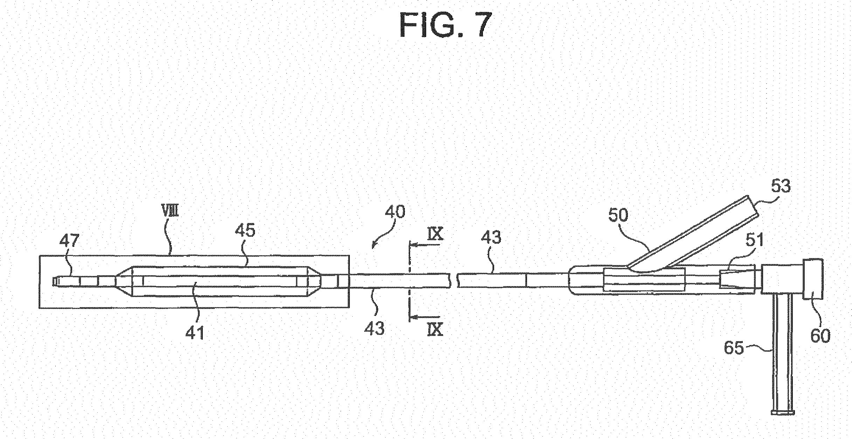

Fig. 7 is a side view of a balloon catheter included in the chemical ablation device illustrated in Fig. 1.

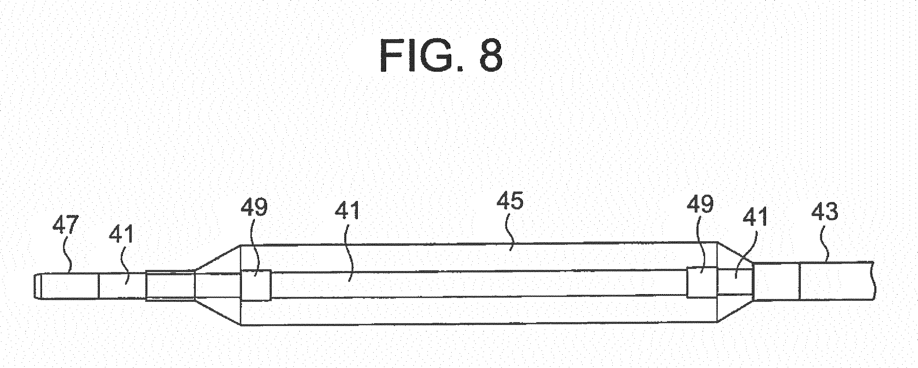

Fig. 8 is an enlargement of a part (a detailed diagram of part VIII) illustrated in Fig. 7.
Fig. 9 is a IX-IX sectional view of Fig. 7.
Fig. 10 is a diagram illustrating the chemical ablation device illustrated in Fig. 1 that is in use.

### Description of Embodiments

An embodiment of the present invention will now be described with reference to the drawings.

A chemical ablation device according to the present embodiment includes: an electrode-equipped guidewire 30 with which intracardiac potential is measurable and that includes a core wire 31, a coil spring 33 provided at a distalmost part of the core wire 31 and extending in the axial direction thereof, a resin shaft 35 that encloses a distal portion of the core wire 31 excluding the distalmost part, a metal shaft 37 connected to the proximal side of the resin shaft 35, a connector 39 connected to the proximal side of the metal shaft 37, electrodes 361 and 362 provided on the distalmost part of the core wire 31, ring-shaped electrodes 363, 364, 365, and 366 each provided around the outer periphery of the resin shaft 35, conductor wires 381 to 386 connected to the electrodes 361 to 366, respectively, and to the connector 39 while running through inside of the resin shaft 35 and inside of the metal shaft 37;
an over-the-wire balloon catheter 40 including an inner tube 41 having a guidewire lumen 415 into which the electrode-equipped guidewire 30 is to be inserted, an outer tube 43 provided on the outer side of the inner tube 41 in such a manner as to provide an expansion lumen 435, a balloon 45 provided on the outer side of the inner tube 41 with the proximal end thereof attached to the distal end of the outer tube 43, and a distal tip 47 that is continuous with the inner tube 41 and extends from the distal end of the balloon 45;
a Y-shaped connector 50 connected to the proximal side of the balloon catheter 40 and including a guidewire port 51 communicating with the guidewire lumen 415 of the balloon catheter 40, and an expansion port 53 communicating with the expansion lumen 435 of the balloon catheter 40; and
a hemostasis valve 60 connected to the guidewire port 51 of the Y-shaped connector 50 and including a side-infusion tube 65 for supplying ethanol as a medical fluid for ablation to the guidewire lumen 415 of the balloon catheter 40,
wherein the inner tube 41 and the distal tip 47 of the balloon catheter 40 have respective inside diameters that provide therebetween a gap serving as an ethanol channel even with the electrode-equipped guidewire 30 (the resin shaft 35) lying in the lumens thereof, and the ethanol supplied to the guidewire lumen 415 is ejected from an opening of the distal tip 47 through the guidewire lumen 415 and through the lumen of the distal tip 47.

The chemical ablation device according to the present embodiment includes the electrode-equipped guidewire 30, the balloon catheter 40, the Y-shaped connector 50, and the hemostasis valve 60.

### <Electrode-Equipped Guidewire 30>

The electrode-equipped guidewire 30 included in the chemical ablation device according to the present embodiment is an electrode catheter having a small diameter and that is usable as a guidewire.

The electrode-equipped guidewire 30 includes the core wire 31, the coil spring 33, the resin shaft 35, the metal shaft (hypo tube) 37, the connector 39, the electrodes 361 to 366, and the conductor wires 381 to 386.

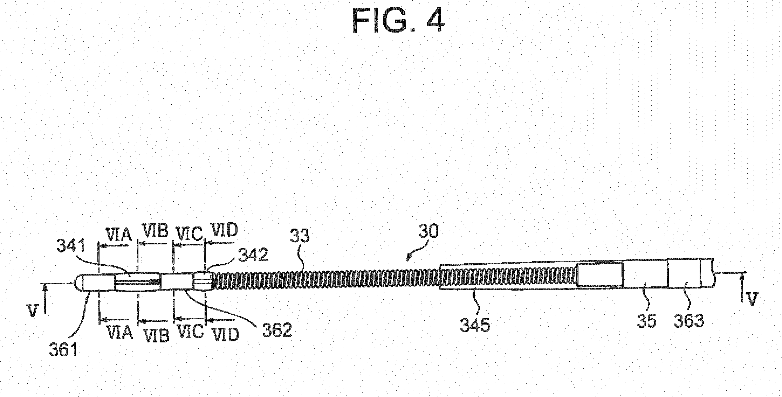

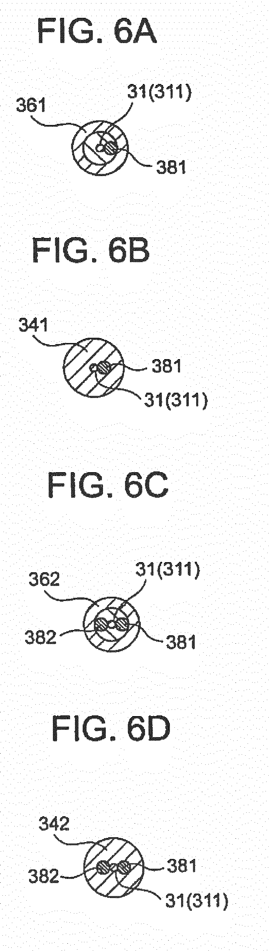

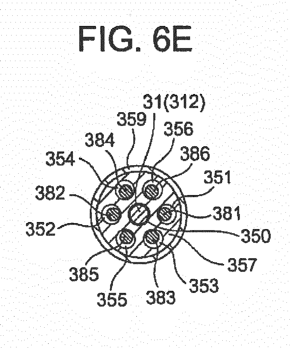

Referring to Figs. 4 and 5 and Figs. 6B and 6D, reference numerals 341 and 342 denote ring-shaped insulating members, respectively, formed from adhesive.

Furthermore, reference numeral 345 denotes a strain relief made of a resin material.

The core wire 31 included in the electrode-equipped guidewire 30 typically includes a distal-side small-diameter portion 311 and a proximal-side large-diameter portion 312 having an outside diameter larger than that of the distal-side small-diameter portion.

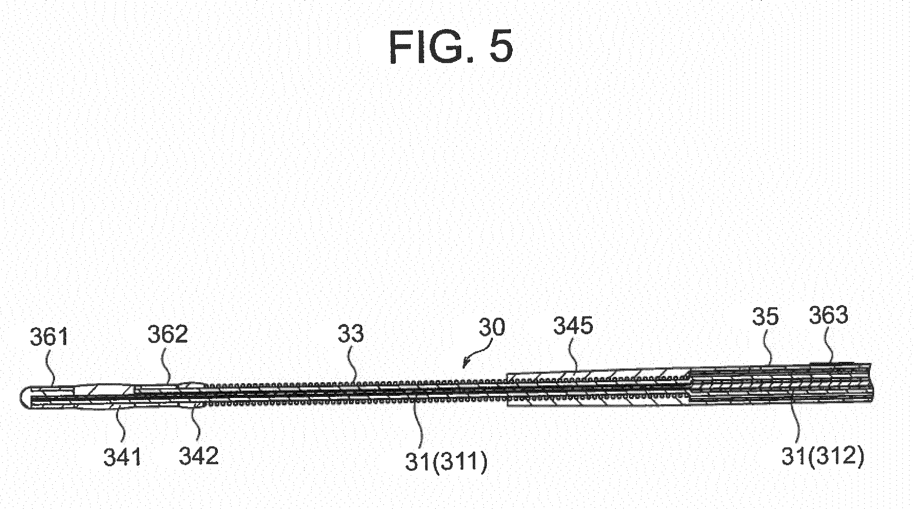

As illustrated in Fig. 5, according to the present embodiment, the distal-side small-diameter portion 311 forms the distalmost part of the core wire 31.

A tapered portion whose outside diameter changes in the axial direction may be provided between the distal-side small-diameter portion 311 and the proximal-side large-diameter portion 312.

The distal end of the core wire 31 is fitted into the inside of the electrode 361 (a distal electrode) and is fixed thereto with solder or the like. The proximal end of the core wire 31 is fixed to, for example, the proximal end of the metal shaft 37.

The distal-side small-diameter portion 311 of the core wire 31 has an outside diameter of, for example, 0.01 to 0.15 mm, or preferably 0.04 to 0.08 mm.

The proximal-side large-diameter portion 312 of the core wire 31 has an outside diameter of, for example, 0.10 to 0.20 mm, or preferably 0.12 to 0.16 mm.

The material forming the core wire 31 is not specifically limited and may be stainless steel (for example, SUS316 or SUS304), gold, platinum, aluminum, tungsten, tantalum, an alloy composed of any of the foregoing materials, Ni-Ti, or the like. The surface of such metal may be coated with resin.

Here, stainless steel can be named as a material suitable for the core wire 31.

The distalmost part (the distal-side small-diameter portion 311) of the core wire 31 is provided with the coil spring 33 extending in the axial direction of the core wire 31.

The coil spring 33 preferably has an outside coil diameter of 0.76 mm or smaller. A suitable example is 0.36 mm (0.014 inches).

The coil spring 33 has a length of, for example, 5 to 30 mm, or preferably 8 to 15 mm.

The material forming the coil spring 33 is not specifically limited and may be stainless steel (for example, SUS316 or SUS304) or the like. The surface of the coil spring 33 may be coated with resin.

The distal end of the coil spring 33 is fixed to the core wire 31 with the adhesive forming the insulating member 342. The proximal end of the coil spring 33 is fixed to core wire 31 and to the distal end of the resin shaft 35 with the resin material forming the strain relief 345.

The core wire 31 and the coil spring 33 configured as above provide the flexibility, the flexural rigidity, and the torque transmissibility that are required as a guidewire, and can satisfy the ease of operation as a guidewire.

The distal portion (the proximal-side large-diameter portion 312) of the core wire 31 excluding the distalmost part and provided with the coil spring 33 is enclosed by the resin shaft 35.

The resin shaft 35 included in the electrode-equipped guidewire 30 has a multi-lumen structure including a center lumen 350 and six sub-lumens 351 to 356 arranged therearound at intervals of 60°.

In the resin shaft 35, the center lumen 350 and the sub-lumens 351 to 356 are provided in an inner portion 357 made of, for example, a low-hardness nylon elastomer.

The outer peripheral surface of the inner portion 357 having the lumens 350 to 356 is covered with an outer portion 359 made of, for example, a high-hardness nylon elastomer.

The resin shaft 35 preferably has an outside diameter of 0.89 mm or smaller so as to be insertable into the guidewire lumen 415 of the balloon catheter 40 and in view of providing an ethanol channel (gap) even with the resin shaft 35 lying therein. A suitable example is 0.64 mm (0.025 inches).

The resin shaft 35 has a length of, for example, 10 to 40 mm, or preferably 15 to 25 mm.

The center lumen 350 provided in the resin shaft 35 has a diameter of, for example, 0.13 to 0.20 mm, or preferably 0.15 to 0.18 mm.

The sub-lumens 351 to 356 provided in the resin shaft 35 each have a diameter of, for example, 0.10 to 0.20 mm, or preferably 0.12 to 0.16 mm.

The proximal-side large-diameter portion 312 of the core wire 31 extends through the center lumen 350 of the resin shaft 35.

A part of the inner portion 357 that is exposed by stripping a part of the outer portion 359 that is at a proximal part of the resin shaft 35 is inserted into an opening at the distal end of the metal shaft 37. Thus, the resin shaft 35 is connected to the metal shaft 37.

The metal shaft 37 included in the electrode-equipped guidewire 30 has a single-lumen structure made of stainless steel, Ni-Ti, a Cu-Mn-Al alloy, or the like.

The connector 39 is connected to the proximal side of the metal shaft 37.

The metal shaft 37 preferably has an outside diameter that is the same as or slightly smaller than the outside diameter of the resin shaft 35.

The metal shaft 37 has a length of, for example, 800 to 2200 mm, or preferably 1200 to 1600 mm.

The core wire 31 is provided with the electrodes 361 and 362 at the distalmost part thereof and at positions on the distal side thereof with respect to the position where the coil spring 33 is provided. The electrodes 361 and 362 are intended for the measurement of the intracardiac potential (bipolar potential).

The electrodes 361 and 362 each have an outside diameter of 0.76 mm or smaller.

Distal parts of the respective conductor wires 381 and 382 are connected to the electrodes 361 and 362, respectively. The conductor wires 381 and 382 extend through the inside of the resin shaft 35 (the sub-lumens 351 and 352, respectively) and through the inside of the metal shaft 37, and respective proximal parts thereof are connected to the connector 39.

The ring-shaped electrodes 363, 364, 365, and 366 for measuring the intracardiac potential are each provided around the outer periphery of the resin shaft 35.

Distal parts of the respective conductor wires 383 to 386 are connected to the ring-shaped electrodes 363 to 366, respectively. The conductor wires 383 to 386 extend through the inside of the resin shaft 35 (the sub-lumens 353 to 356, respectively) and through the inside of the metal shaft 37, and are connected to the connector 39 at respective proximal parts thereof.

### <Balloon Catheter 40>

The balloon catheter 40 included in the chemical ablation device according to the present embodiment is an over-the-wire balloon catheter including the inner tube 41, the outer tube 43, the balloon 45, and the distal tip 47.

The inner tube 41 included in the balloon catheter 40 has the guidewire lumen 415 having an inside diameter that allows the insertion of the electrode-equipped guidewire 30 therethrough.

The guidewire lumen 415 serves as an insertion path for the electrode-equipped guidewire 30 and as a channel for the ethanol serving as a medical fluid for ablation.

Contrast markers 49 are each provided around the outer periphery of the inner tube 41 (at two respective positions) on the inside of the balloon 45.

The inner tube 41 preferably has an outside diameter of 0.60 to 1.10 mm. A suitable example is 0.85 mm.

The inner tube 41 preferably has an inside diameter (a diameter of the guidewire lumen 415) of 0.68 mm or larger, or more preferably 0.70 to 1.00 mm, in view of providing an ethanol channel even with the resin shaft 35 of the electrode-equipped guidewire 30 lying therein. A suitable example is 0.75 mm.

The material forming the inner tube 41 may be synthetic resin such as polyolefin, polyamide, polyether polyamide, polyurethane, nylon, or PEBAX (a registered trademark) (polyether block amide). Among the forgoing materials, PEBAX is preferable.

The outer tube 43 included in the balloon catheter 40 has the expansion lumen 435 that provides a channel through which a fluid for expanding the balloon 45 flows.

Here, a physiological saline solution can be named as an exemplary fluid to be supplied to the expansion lumen 435.

The outer tube 43 preferably has an outer diameter of 1.00 to 1.40 mm. A suitable example is 1.20 mm.

The outer tube 43 preferably has an inside diameter of 0.80 to 1.20 mm. A suitable example is 1.00 mm.

The outer tube 43 preferably has a length of 700 to 1300 mm. A suitable example is 1000 mm.

The material forming the outer tube 43 may be any of the synthetic resins listed above for the inner tube 41. In particular, PEBAX is preferable.

The balloon 45 included in the balloon catheter 40 typically has an expanded diameter of 1.0 to 3.0 mm, or preferably 1.5 to 2.5 mm.

The balloon 45 typically has a length of 5 to 30 mm, or preferably 10 to 20 mm.

The material forming the balloon 45 may be the same as any of the materials forming balloons of publicly known balloon catheters. PEBAX can be named as a suitable material.

The distal tip 47 included in the balloon catheter 40 is continuous with the inner tube 41 and extends from the distal end of the balloon 45.

The distal tip 47 has an outside diameter and an inside diameter that are the same as the outside diameter and the inside diameter (the diameter of the guidewire lumen 415), respectively, of the inner tube 41.

The inside diameter (an opening diameter) of the distal tip 47 is larger than the outside diameter of the resin shaft 35 included in the electrode-equipped guidewire 30, with a preferable difference therebetween of 0.05 to 0.50 mm.

The distal tip 47 preferably has a length of 1 to 10 mm. A suitable example is 3 mm.

The distal tip 47 may have a plurality of side holes around the outer periphery thereof. In that case, the medical fluid can also be fed to a site that is on the proximal side with respect to the openings of the distal tip 47, whereby that site can also be chemically ablated (cauterized).

### <Y-Shaped Connector 50>

The Y-shaped connector 50 is attached to the proximal side of the balloon catheter 40.

The Y-shaped connector 50 included in the chemical ablation device according to the present embodiment includes the guidewire port 51 communicating with the guidewire lumen 415 of the balloon catheter 40, and the expansion port 53 communicating with the expansion lumen 435 of the balloon catheter 40.

### <Hemostasis Valve 60>

The hemostasis valve 60 is attached to the guidewire port 51 of the Y-shaped connector 50.

The electrode-equipped guidewire 30 is inserted from the guidewire port 51 of the Y-shaped connector 50 into the guidewire lumen 415 of the balloon catheter 40 through the hemostasis valve 60.

The hemostasis valve 60 included in the chemical ablation device according to the present embodiment includes the side-infusion tube 65. Ethanol as a medical fluid for ablation is supplied from the side-infusion tube 65.

The ethanol supplied from the side-infusion tube 65 into the hemostasis valve 60 flows from the guidewire port 51 of the Y-shaped connector 50 into the guidewire lumen 415 of the balloon catheter 40 and into the lumen of the distal tip 47, and is ejected from the opening of the distal tip 47.

### <Chemical Ablation System>

A chemical ablation system according to the present embodiment includes a primary guiding catheter, a secondary guiding catheter insertable into a lumen of the primary guiding catheter, and the above-described chemical ablation device, the device including an electrode-equipped guidewire including a portion that is insertable into a lumen of the secondary guiding catheter, and a balloon catheter.

The primary guiding catheter included in the chemical ablation system according to the present embodiment typically has an outside diameter of 2.4 to 3.3 mm, or preferably 2.9 to 3.1 mm.

The primary guiding catheter typically has an inside diameter of 1.9 to 2.8 mm, or preferably 2.2 to 2.5 mm.

The secondary guiding catheter included in the chemical ablation system according to the present embodiment has an outside diameter that is smaller than the inside diameter of the primary guiding catheter and that is typically 1.8 to 2.5 mm, or preferably 2.1 to 2.3 mm.

The secondary guiding catheter typically has an inside diameter of 1.5 to 2.2 mm, or preferably 1.7 to 1.9 mm.

Using the chemical ablation device (system) according to the present embodiment, a method of ethanol infusion in the vein of Marshall (VOM) can be performed in the following procedure.

Specifically, the electrode-equipped guidewire 30 is inserted and the potential is measured, the balloon catheter 40 is inserted into the vein of Marshall along the electrode-equipped guidewire 30, and ethanol is flashed from the gap between the opening of the distal tip 40 of the balloon catheter 40 and the resin shaft 35 of the electrode-equipped guidewire 30 without removing the electrode-equipped guidewire 30 therefrom.

Details will now be described.

### (1) Insertion of Primary Guiding Catheter:

First, approaching from the superior vena cava, the distal end of a primary guiding catheter 10 is made to engage with the entrance of the coronary sinus (CS).

In this step, the following operation may be performed so that the presence/absence of the vein of Marshall (VOM) is checked: An over-the-wire balloon catheter (a balloon catheter different from the one included in the chemical ablation system according to the present embodiment) is inserted into a lumen of the primary guiding catheter 10, and a balloon is expanded at the entrance of the coronary sinus (CS) for occlusion. Simultaneously, a contrast medium is ejected from a distal tip of the balloon catheter, whereby a contrast image of the coronary sinus (CS) is taken.

### (2) Insertion of Secondary Guiding Catheter:

Subsequently, a secondary guiding catheter 20 is inserted into the lumen of the primary guiding catheter 10, and the distal end of the secondary guiding catheter 20 that is made to project from an opening at the distal end of the primary guiding catheter 10 is positioned near the entrance of the vein of Marshal (VOM).

### (3) Insertion of Electrode-Equipped Guidewire 30 (Pre-Treatment Potential Measurement)

In advance, the electrode-equipped guidewire 30 is inserted into the guidewire lumen 415 of the balloon catheter 40 and into the lumen of the distal tip 47, and the distal end of the electrode-equipped guidewire 30 is drawn out of the opening of the distal tip 47. With the two connected to each other in such a manner, the electrode-equipped guidewire 30 is operated.

The electrode-equipped guidewire 30 and the balloon catheter 40 are inserted into the lumen of the secondary guiding catheter 20, and the distal portion (the portion provided with the ring-shaped electrodes 361 to 366) of the electrode-equipped guidewire 30 that is made to project from the opening at the distal end of the secondary guiding catheter 20 is inserted into the vein of Marshal (VOM). Then, a pre-treatment potential measurement (pacing/mapping) is performed.

The distal end of the electrode-equipped guidewire 30 has a performance level equivalent to that of the distal end of a typical guidewire for medical use. Therefore, the distal end thereof can be shaped into a curved or any other like form.

Moreover, since the electrode-equipped guidewire 30 can be operated in the same manner as the typical guidewire for medical use, the electrode-equipped guidewire 30 can be introduced into the vein of Marshal relatively easily.

### (4) Insertion of Balloon Catheter 40 and Ablation Treatment:

Subsequently, the inner tube 41 is passed through the electrode-equipped guidewire 30, whereby the balloon catheter 40 is inserted into the vein of Marshal (VOM) along the electrode-equipped guidewire 30.

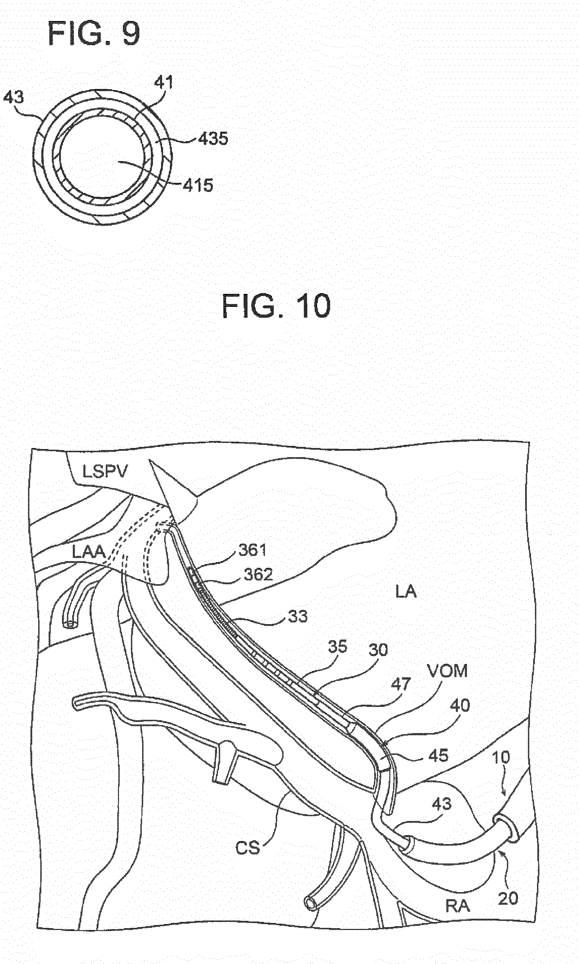

Fig. 10 illustrates the balloon catheter 40 projecting from the opening at the distal end of the secondary guiding catheter 20 and that is being inserted into the vein of Marshal (VOM) along the electrode-equipped guidewire 30.

In the state illustrated in Fig. 10, the distal portion of the resin shaft 35 included in the electrode-equipped guidewire 30 projects from the opening of the distal tip 47 included in the balloon catheter 40.

Subsequently, in the state illustrated in Fig. 10, that is, with the electrode-equipped guidewire 30 lying in the vein of Marshal (VOM), the balloon 45 of the balloon catheter 40 is expanded. Then, ethanol is infused from the side-infusion tube 65.

The ethanol thus infused flows from the hemostasis valve 60 through the inner tube 41 (the guidewire lumen 415) of the balloon catheter 40, in which the electrode-equipped guidewire 30 is lying, and through the lumen of the distal tip 47, and is infused into the vein of Marshal (VOM) from the gap between the opening of the distal tip 47 and the resin shaft 35. Thus, chemical ablation is performed.

Usually, chemical ablation (ethanol infusion) starts to be performed from the far side where capillary vessels are present, then chemical ablation is performed multiple times (in three to four times) by moving the balloon catheter stepwise towards the entrance.

### (5) Post-Treatment Potential Measurement:

Subsequently, a post-treatment potential measurement (pacing/mapping) is performed by using the electrode-equipped guidewire 30 that is still lying in the balloon catheter 40 (the inner tube 41).

If the result of the potential measurement shows that the ablation is insufficient, the treatment can be restarted immediately.

The chemical ablation device and the chemical ablation system according to the present embodiment enable simple performance of a procedure associated with a method of ethanol infusion in the vein of Marshall including pre- and post-treatment potential measurements.

### Reference Signs List

- 10: primary guiding catheter
- 20: secondary guiding catheter
- 30: electrode-equipped guidewire
- 31: core wire
- 311: distal-side small-diameter portion of core wire
- 312: proximal-side large-diameter portion of core wire
- 33: coil spring
- 341, 342: insulating member
- 345: strain relief
- 35: resin shaft
- 350: center lumen
- 351 to 356: sub-lumen
- 357: inner portion
- 359: outer portion
- 361, 362: electrode
- 363 to 366: ring-shaped electrode
- 381 to 386: conductor wire
- 37: metal shaft
- 39: connector
- 40: balloon catheter
- 41: inner tube
- 415: guidewire lumen
- 43: outer tube
- 435: expansion lumen
- 45: balloon
- 47: distal tip
- 50: Y-shaped connector
- 51: guidewire port
- 53: expansion port
- 60: hemostasis valve
- 65: side-infusion tube

## Claims

1. A chemical ablation device comprising:
an electrode-equipped guidewire (30) with which intracardiac potential is measurable;
an over-the-wire balloon catheter (40) including an inner tube (41) having a guidewire lumen (415) into which the electrode-equipped guidewire (30) is to be inserted, an outer tube (43) provided on an outer side of the inner tube (41) in such a manner as to provide an expansion lumen (435), a balloon (45) provided on the outer side of the inner tube (41) with a proximal end thereof attached to a distal end of the outer tube (43), and a distal tip (47) that is continuous with the inner tube (41) and extends from a distal end of the balloon (45);
a Y-shaped connector (50) connected to a proximal side of the balloon catheter (40) and including a guidewire port (51) communicating with the guidewire lumen (415) of the balloon catheter (40), and an expansion port (53) communicating with the expansion lumen (435) of the balloon catheter (40); and
a hemostasis valve (60) connected to the guidewire port (51) of the Y-shaped connector (50) and including a side-infusion tube (65) for supplying a medical fluid for ablation to the guidewire lumen (415) of the balloon catheter (40),
wherein the medical fluid supplied to the guidewire lumen (415) of the balloon catheter (40) is ejected from an opening of the distal tip (47).

2. The chemical ablation device according to Claim 1, wherein the electrode-equipped guidewire (30) includes a core wire (31), a resin shaft (35) that encloses at least a distal portion of the core wire (31) excluding a distalmost part of the core wire (31), a connector (39) connected to a proximal side of the resin shaft (35) with or without a metal shaft (37) in between, a plurality of ring-shaped electrodes (363, 364, 365, 366) each provided around an outer periphery of the resin shaft (35), and a plurality of conductor wires (381, 382, 383, 384, 385, 386) connected to the ring-shaped electrodes (363, 364, 365, 366), respectively, and to the connector (39) while running through an inside of the resin shaft (35).

3. The chemical ablation device according to Claim 2, wherein the electrode-equipped guidewire (30) includes a coil spring (33) attached to the distalmost part of the core wire (31) and extending in an axial direction of the core wire (31).

4. The chemical ablation device according to Claim 3, wherein the electrode-equipped guidewire (30) is provided with an electrode (361) on a distal side of the distalmost part of the core wire (31) with respect to a position where the coil spring (33) is provided.

5. The chemical ablation device according to Claim 3 or 4,
wherein an outside diameter of the electrode (361) provided on the distalmost part of the core wire (31) of the electrode-equipped guidewire (30) and an outside coil diameter of the coil spring (33) of the electrode-equipped guidewire (30) are each 0.76 mm or smaller, and
wherein the resin shaft (35) has an outside diameter of 0.89 mm or smaller.

6. The chemical ablation device according to Claim 5,
wherein a difference between an opening diameter of the distal tip (47) included in the balloon catheter (40) and the outside diameter of the resin shaft (35) included in the electrode-equipped guidewire 830) is 0.05 to 0.50 mm, and
wherein, in a state where the distal portion of the resin shaft (35) is made to project from the opening of the distal tip (47), the medical fluid is ejected from a gap between the distal tip (47) and the resin shaft (35).

7. The chemical ablation device according to any of Claims 1 to 6, wherein the distal tip (47) included in the balloon catheter (40) has a plurality of side holes on an outer periphery thereof.

8. A chemical ablation system comprising:
a primary guiding catheter having an inside diameter of 1.9 to 2.6 mm;
a secondary guiding catheter that is insertable into a lumen of the primary guiding catheter and has an inside diameter of 1.5 to 2.2 mm; and
the chemical ablation device according to Claim 1.

## Patentansprüche

1. Vorrichtung zur chemischen Ablation, umfassend:
einen mit Elektroden ausgestatteten Führungsdraht (30), mit dem ein intrakardiales Potential messbar ist;
einen Over-the-Wire Ballonkatheter (40), umfassend einen inneren Schlauch (41) mit einem Führungsdraht-Lumen (415), in welches der mit Elektroden ausgestattete Führungsdraht (30) einzuführen ist, einen äußeren Schlauch (43), der an einer äußeren Seite des inneren Schlauchs (41) derart vorgesehen ist, dass er ein Expansions-Lumen (435) bereitstellt, einen Ballon (45), der an der äußeren Seite des inneren Schlauchs (41) vorgesehen ist, wobei ein proximales Ende von ihm an einem distalen Ende des äußeren Schlauchs (43) befestigt ist, und eine distale Spitze (47), die kontinuierlich mit dem inneren Schlauch (41) ist und sich von einem distalen Ende des Ballons (45) erstreckt;
ein Y-förmiges Verbindungsstück (50), das mit einer proximalen Seite des Ballonkatheters (40) verbunden ist, und einen Führungsdraht-Anschluss (51), der mit dem Führungsdraht-Lumen (415) des Ballonkatheters (40) in Verbindung steht, und einen Expansions-Anschluss (53), der mit dem Expansions-Lumen (435) des Ballonkatheters (40) in Verbindung steht, umfasst; und
ein Hämostase-Ventil (60), das mit dem Führungsdraht-Anschluss (51) des Y-förmigen Verbindungsstücks (50) verbunden ist, und einen Seiteninfusionsschlauch (65) zum Zuführen einer medizinischen Flüssigkeit zur Ablation zu dem Führungsdraht-Lumen (415) des Ballonkatheters (40) umfasst,
wobei die medizinische Flüssigkeit die dem Führungsdraht-Lumen (415) des Ballonkatheters zugeführt wird, aus einer Öffnung der distalen Spitze (47) ausgestoßen wird.

2. Vorrichtung zur chemischen Ablation nach Anspruch 1, wobei der mit Elektroden ausgestattete Führungsdraht (30) einen Kerndraht (31), einen Harzschaft (35), der zumindest einen distalen Abschnitt des Kerndrahts (31) ausschließlich eines distalsten Teils des Kerndrahts (31) einkapselt, ein Verbindungsstück (39), das mit einer proximalen Seite des Harzschafts (35) mit oder ohne einen dazwischenliegenden Metallschaft (37) verbunden ist, eine Vielzahl von ringförmigen Elektroden (363, 364, 365, 366), die jeweils um einen äußeren Umfang des Harzschafts (35) herum vorgesehen sind, und eine Vielzahl von Leitungsdrähten (381, 382, 383, 384, 385, 386), die jeweils mit den ringförmigen Elektroden (363, 364, 365, 366) und dem Verbindungsstück (39) verbunden sind, wobei sie durch ein Inneres des Harzschafts (35) verlaufen, umfasst.

3. Vorrichtung zur chemischen Ablation nach Anspruch 2, wobei der mit Elektroden ausgestattete Führungsdraht (30) eine Spiralfeder (33) umfasst, die mit dem distalsten Teil des Kerndrahts (31) verbunden ist und sich in einer axialen Richtung des Kerndrahts (31) erstreckt.

4. Vorrichtung zur chemischen Ablation nach Anspruch 3, wobei der mit Elektroden ausgestattete Führungsdraht (30) mit einer Elektrode (361) auf einer bezüglich einer Position, an der die Spiralfeder (33) vorgesehen ist, distalen Seite des distalsten Teils des Kerndrahts (31), versehen ist.

5. Vorrichtung zur chemischen Ablation nach Anspruch 3 oder Anspruch 4,
wobei ein Außendurchmesser der Elektrode (361), die an dem distalsten Teil des Kerndrahts (31) des mit Elektroden ausgestatteten Führungsdrahts (30) vorgesehen ist, und ein äußerer Windungsdurchmesser der Spiralfeder (33) des mit Elektroden ausgestatteten Führungsdrahts (30) jeweils 0,76 mm oder kleiner ist, und
wobei der Harzschaft (35) einen Außendurchmesser von 0,89 mm oder kleiner hat.

6. Vorrichtung zur chemischen Ablation nach Anspruch 5,
wobei eine Differenz zwischen einem Öffnungsdurchmesser der in dem Ballonkatheter (40) enthaltenen distalen Spitze (47) und dem Außendurchmesser des in dem mit Elektroden ausgestatteten Führungsdrahts 830) 0,05 bis 0,50 mm beträgt, und
wobei in einem Zustand, in dem der distale Abschnitt des Harzschafts (35) aus der Öffnung der distalen Spitze (47) herausragt, die medizinische Flüssigkeit aus einem Spalt zwischen der distalen Spitze (47) und dem Harzschaft (35) ausgestoßen wird.

7. Vorrichtung zur chemischen Ablation nach einem der Ansprüche 1 bis 6, wobei die in dem Ballonkatheter (40) enthaltene distale Spitze (47) eine Vielzahl von seitlichen Löchern auf einem äußeren Umfang von ihr hat.

8. System zur chemischen Ablation, umfassend:
einen primären Führungskatheter mit einem Innendurchmesser zwischen 1,9 und 2,6 mm;
einen sekundären Führungskatheter, der in ein Lumen des primären Führungskatheters einführbar ist und einen Innendurchmesser zwischen 1,5 und 2 mm hat; und
die Vorrichtung zur chemischen Ablation nach Anspruch 1.

## Revendications

1. Dispositif d'ablation chimique comprenant :
un fil guide équipé d'une électrode (30) avec lequel un potentiel intracardiaque peut être mesuré ;
un cathéter à ballonnet sur le fil (40) comprenant un tube interne (41) ayant une lumière de fil guide (415) dans laquelle le fil guide équipé d'une électrode (30) doit être inséré, un tube externe (43) disposé sur un côté externe du tube interne (41) de manière à fournir une lumière d'expansion (435), un ballonnet (45) disposé sur le côté externe du tube interne (41) avec son extrémité proximale fixée à une extrémité distale du tube externe (43), et une pointe distale (47) qui est continue avec le tube interne (41) et qui s'étend depuis une extrémité distale du ballonnet (45) ;
un connecteur en forme de Y (50) relié à une extrémité proximale du cathéter à ballonnet (40) et comprenant un orifice fil guide (51) communiquant avec la lumière du fil guide (415) du cathéter à ballonnet (40), et un orifice d'expansion (53) communiquant avec la lumière d'expansion (435) du cathéter à ballonnet (40) ; et
une valve d'hémostase (60) reliée à l'orifice fil guide (51) du connecteur en forme de Y (50) et comprenant un tube de perfusion latéral (65) pour alimenter un liquide médical pour l'ablation à la lumière du fil guide (415) du cathéter à ballonnet (40),
où le liquide médical alimenté à la lumière du fil guide (415) du cathéter à ballonnet (40) est éjecté d'une ouverture de la pointe distale (47).

2. Dispositif d'ablation chimique selon la revendication 1, le fil guide équipé d'une électrode (30) comprenant un fil central (31), une tige de résine (35) qui enferme au moins une partie distale du fil central (31) excluant une partie la plus distale du fil central (31), un connecteur (39) relié à un côté proximal de la tige de résine (35) avec ou sans tige métallique (37) entre eux, une pluralité d'électrodes en forme d'anneau (363, 364, 365, 366) disposées chacune autour d'une périphérie externe de la tige de résine (35), et une pluralité de fils conducteurs (381, 382, 383, 384, 385, 386) reliés aux électrodes en forme d'anneau (363, 364, 365, 366), respectivement, et au connecteur (39) tout en courant à travers un intérieur de la tige de résine (35).

3. Dispositif d'ablation chimique selon la revendication 2, le fil guide équipé d'une électrode (30) comprenant un ressort spiralé (33) fixé à la partie la plus distale du fil central (31) et s'étendant dans un sens axial du fil central (31).

4. Dispositif d'ablation chimique selon la revendication 3, le fil guide équipé d'une électrode (30) étant muni d'une électrode (361) sur un côté distal de la partie la plus distale du fil central (31) par rapport à une position où le ressort spiralé (33) est disposé.

5. Dispositif d'ablation chimique selon la revendication 3 ou 4,
dans lequel le diamètre externe de l'électrode (361) disposé sur la partie la plus distale du fil central (31) du fil guide équipé d'une électrode (30) et d'un diamètre de bobine extérieur du ressort spiralé (33) du fil guide équipé d'une électrode (30) sont chacun de 0,76 mm ou moins, et
la tige de résine (35) présente un diamètre externe de 0,89 mm ou moins.

6. Dispositif d'ablation chimique selon la revendication 5,
dans lequel une différence entre un diamètre d'ouverture de la pointe distale (47) comprise dans le cathéter à ballonnet (40) et le diamètre externe de la tige de résine (35) comprise dans le fil guide équipé d'une électrode (30) est de 0,05 à 0,50 mm, et
où, dans un état où la partie distale de la tige de résine (35) est constituée pour se projeter depuis l'ouverture de la pointe distale (47), le liquide médical est éjecté depuis un espace entre la pointe distale (47) et la tige de résine (35).

7. Dispositif d'ablation chimique selon l'une quelconque des revendications 1 à 6, dans lequel la pointe distale (47) comprise dans le cathéter à ballonnet (40) présente une pluralité de trous latéraux sur sa périphérie externe.

8. Système d'ablation chimique comprenant :
un cathéter de guidage principal ayant un diamètre intérieur de 1,9 à 2,6 mm ;
un cathéter de guidage secondaire qui peut être inséré dans une lumière du cathéter de guidage primaire et présente un diamètre intérieur de 1,5 à 2,2 mm ;
et le dispositif d'ablation chimique selon la revendication 1.
